Europäisches Patentamt

European Patent Office

Office européen des brevets

(11) Numéro de publication: **0 291 411 B1**

# FASCICULE DE BREVET EUROPEEN

(45) Date de publication de fascicule du brevet: **15.04.92**

(51) Int. Cl.5: **C07C 11/113**, C07C 2/24, B01J 27/232

(21) Numéro de dépôt: **88401157.8**

(22) Date de dépôt: **11.05.88**

(54) **Catalyseur et procédé de dimérisation du propylène en méthyl-4-pentène-1.**

(30) Priorité: **15.05.87 FR 8706828**

(43) Date de publication de la demande:
**17.11.88 Bulletin 88/46**

(45) Mention de la délivrance du brevet:
**15.04.92 Bulletin 92/16**

(84) Etats contractants désignés:
**AT BE CH DE ES FR GB GR IT LI LU NL SE**

(56) Documents cités:
**EP-A- 0 207 378**
**FR-A- 2 200 047**
**US-A- 2 390 100**
**US-A- 3 816 555**

**BULLETIN OF THE CHEMICAL SOCIETY OF JAPAN, vol. 48, no. 4, avril 1975, pages 1272-1275; J. YAMASHITA et al.: "Selective dimerization of propene to 4-methyl-1-pentene by potassium-copper based catalysts"**

**CHEMICAL ABSTRACTS, vol. 80, no. 21, 27 mai 1974, page 378, résumé no. 120195r, Columbus, Ohio, US; & JP-A-73 37 241**

(73) Titulaire: **ATOCHEM**
**4 & 8, Cours Michelet La Défense 10**
**F-92800 Puteaux(FR)**

(72) Inventeur: **Fuchs, Jean-Marc**
**34, rue Roger Salengro**
**F-62172 Bouvigny Boyeffles(FR)**
Inventeur: **Gallot, Michel**
**19, rue Basse Boulogne**
**F-62149 Cambrin(FR)**
Inventeur: **Saussine, Lucien**
**2, Place des Frères Tissandier**
**F-78290 Croissy sur Seine(FR)**

(74) Mandataire: **Rochet, Michel et al**
**ATOCHEM Département Propriété Industriel-le 4, Cours Michelet La Défense 10 Cedex 42**
**F-92091 Paris-La-Défense(FR)**

## Description

Il est déjà connu de dimériser le propylène en méthyl-4-pentène-1 au moyen de catalyseurs comprenant un ou plusieurs métaux.

Ainsi le document US-A-2.994.725 décrit un procédé de dimérisation de mono-oléfines à une température de 100 à 350°C sous une pression de 5 à 100 bars en présence d'une quantité catalytique d'un métal alcalin et de fer finement divisé agissant comme promoteur.

Le document US-A-3.755.491 décrit un procédé de dimérisation du propylène en méthyl-4-pentène-1 en présence d'un catalyseur composé de potassium, de cuivre et d'alcoolate de potassium.

Le document US-A-4.544.790 décrit un procédé de dimérisation des oléfines utilisant un système catalytique comprenant au moins un métal alcalin, du carbonate de potassium et au moins un métal ou alliage choisi parmi le cuivre, le cobalt et l'acier inoxydable.

Le document FR-A-2.200.047 décrit un procédé de préparation d'un catalyseur utilisé dans la dimérisation du propylène en méthyl-4-pentène-1.

Ce catalyseur est préparé sous un courant de gaz inerte en mélangeant des particules de carbonate de potassium avec du sodium ou du potassium et un composé décomposable thermiquement d'un métal de transition choisi parmi l'or, le nickel, le chrome, le palladium, le cobalt et le tungstène, puis en chauffant le mélange ainsi obtenu à une température d'environ 150 à 500°C. Ce composé décomposable thermiquement est choisi de préférence parmi les sels, les oxydes ou les hydroxydes de ces métaux de transition qui se décomposent au-dessous d'une température de l'ordre de 400°C.

On a maintenant trouvé qu'en associant certains autres métaux de transition et, le cas échéant, du cuivre, à des catalyseurs comprenant par ailleurs du potassium et du carbonate de potassium, il était possible de dimériser du propylène en méthyl-4-pentène-1 avec une conversion et/ou une sélectivité améliorée.

Un objet de la présente invention consiste en un catalyseur de dimérisation du propylène en méthyl-4-pentène-1 comprenant du potassium (K) et du carbonate de potassium ($K_2CO_3$), caractérisé en ce qu'il contient en outre au moins un métal (M) choisi parmi le manganèse et le cadmium.

Selon un mode de réalisation préféré de l'invention, le catalyseur comprend, en outre, du cuivre.

Le potassium, les métaux (M) et le cuivre sont à l'état élémentaire dans le catalyseur selon l'invention.

Le carbonate de potassium, jouant le rôle de support pour le potassium, le métal (M) et le cas échéant le cuivre, sont sous la forme de poudre ou de granulés convenablement séchés.

Avantageusement le rapport pondéral (M)/K est compris entre 0,4 et 5.

Avantageusement encore le rapport pondéral Cu/(M) est au plus égal à 2.

Le métal (M) et, le cas échéant, le cuivre, sont avantageusement présents dans le catalyseur sous la forme de poudre ayant une granulométrie comprise entre 30 et 300 $\mu$m, et de préférence comprise entre 50 et 200 $\mu$m.

Le catalyseur selon l'invention peut être préparé par tout moyen conduisant à un mélange intime de ses constituants.

On peut ainsi, après avoir déshydraté le support de carbonate de potassium à une température d'au moins 150 à 200°C, de préférence sous atmosphère inerte et éventuellement par application du vide, ajouter le potassium et le mélanger, à une température supérieure à son point de fusion, sous agitation intense, au carbonate de potassium.

Ensuite le ou les métaux, et le cas échéant le cuivre, sous forme pulvérulente sont incorporés au mélange obtenu sous agitation intense. On obtient ainsi un mélange homogène à l'état solide où le potassium et le métal (M), et le cas échéant le cuivre, sont intimement mélangés au support de carbonate de potassium.

On a trouvé avantageux, afin d'éviter les inconvénients dûs à la manipulation de potassium à l'état fondu dans des malaxeurs de particules solides, d'utiliser le procédé suivant : on opère en présence d'un liquide inerte à haut point d'ébullition selon la séquence d'étapes suivantes :

- dans une première étape on réalise une suspension de carbonate de potassium dans le liquide inerte,
- dans une deuxième étape on introduit dans le milieu réactionnel fortement agité le ou les métaux (M), et le cas échéant du cuivre, sous forme pulvérulente,
- dans une troisième étape, après avoir porté le milieu réactionnel à une température supérieure à la température de fusion du potassium, on introduit le potassium sous forte agitation,
- dans une quatrième étape on porte le milieu réactionnel à une température comprise entre 140 et 180°C pendant une durée comprise entre 1 et 3 heures, puis on le laisse refroidir à température ambiante.

Par liquide inerte à haut point d'ébullition on entend tout liquide ne réagissant avec aucun des constituants du catalyseur, qui n'est pas solvant desdits constituants et qui a un point d'ébullition compris entre 80 et 300°C sous pression atmosphérique. Il peut s'agir d'un hydrocarbure aliphatique ou cycloaliphatique saturé, ramifié ou non, ayant entre 6 et 15 atomes de carbone. On utilise avantageusement une fraction d'hydrocarbures saturés bouillant, sous pression atmosphérique, entre 150 et 250°C.

Le carbonate de potassium est préalablement séché, en vue de le déshydrater, à haute température et, de préférence, sous courant de gaz inerte. Il est également possible d'utiliser un vide au moins partiel pour cette opération. La suspension de carbonate de potassium dans le liquide inerte est préparée à l'aide de tout moyen procurant une agitation intense, de préférence une plaque vibrante. Au cours de la première étape le milieu peut être maintenu à température ambiante ou être légèrement chauffé. Il en est de même au cours de la deuxième étape. Le ou les métaux (M) introduits alors, et le cas échéant le cuivre, sont sous forme pulvérulente ayant avantageusement une granulométrie comprise entre 30 et 300 $\mu$m, de préférence entre 50 et 200 $\mu$m.

Le milieu réactionnel, après la deuxième étape, est porté à une température comprise entre 70 et 150°C, de préférence entre 80 et 120°C, pendant une durée comprise entre 5 et 30 minutes et on ajoute ensuite le potassium sous agitation intense.

Dans la quatrième étape l'agitation est maintenue, de préférence jusqu'à complet refroidissement.

Un ou plusieurs additifs peuvent être introduits lors de la préparation du catalyseur pour, par exemple, améliorer la dispersion des constituants et/ou augmenter la stabilité de la suspension en évitant sa sédimentation.

La suspension obtenue est facilement manipulable et est introduite en l'état dans l'installation de dimérisation au moyen de dispositifs d'introduction connus (injecteurs par aspiration, pompes). Si on le souhaite, le catalyseur selon l'invention peut être récupéré à l'état pâteux ou solide à partir de cette suspension, par exemple par évaporation du liquide inerte sous pression réduite.

Un autre object de la présente invention consiste en un procédé de dimérisation du propylène en méthyl-4-pentène-1 caractérisé en ce qu'il consiste à utiliser comme catalyseur au moins un catalyseur tel que défini ci-dessus. Le catalyseur peut être à l'état solide ou sous forme de suspension dans un liquide inerte.

La dimérisation peut être réalisée selon un mode discontinu ou continu, à une température avantageusement comprise entre 150 et 220°C, sous une pression comprise de préférence entre 2 et 300 bars, de préférence entre 40 et 170 bars et pendant une durée comprise de préférence entre 1 min. et 10 heures, dépendant de la température, de la pression et de l'activité du catalyseur utilisé.

Le catalyseur peut être utilisé dans le procédé de dimérisation selon l'invention selon des teneurs très variées vis-à-vis du propylène. Avantageusement le rapport molaire propylène/potassium est compris entre 50 et 300.

Après réaction, le mélange réactionnel est traité de façon connue, par exemple par distillation fractionnée, dans le but de récupérer le méthyl-4-pentène-1 formé. A côté du méthyl-4-pentène-1, qui est le produit principal visé par l'invention, il peut se former soit par dimérisation soit par isomérisation d'autres composés tels que le méthyl-4-pentène-2, princique le méthyl-2-pentène-1, le méthyl-2-pentène-2, l'hexène-1, l'hexène-2, le cas échéant, pour certains d'entre eux, sous chacune des formes cis et trans.

Il est bien connu que, pour bon nombre de réactions catalysées pouvant conduire à un mélange de produits, le taux de conversion et la sélectivité en produit recherché sont antagonistes. Si l'on désire augmenter le taux de conversion, bien souvent la sélectivité diminue, et réciproquement.

On a constaté que les catalyseurs selon l'invention permettent, à taux de conversion pratiquement inchangé, d'améliorer la sélectivité en méthyl-4-pentène-1. Et réciproquement, en conservant un taux de sélectivité donné, ils permettent d'augmenter la conversion. Avec certains des catalyseurs selon l'invention il a même été trouvé possible d'augmenter en même temps le taux de conversion et la sélectivité en méthyl-4-pentène-1, ceci étant le reflet d'un rendement en méthyl-4-pentène-1 nettement amélioré.

Les exemples ci-après ont pour but d'illustrer l'invention.

## EXEMPLES

### A) Préparation du catalyseur

Avant utilisation, le carbonate de potassium est chauffé sous courant d'azote d'abord à 100°C pendant 4 h 30 puis à 250°C pendant 2 h de façon à ramener sa teneur finale en eau à moins de 0,3 % en poids.

Dans un réacteur purgé à l'azote on introduit 150 ml d'une coupe d'hydrocarbures saturés en $C_{12}$-$C_{14}$ et 50 g de carbonate de potassium. Le mélange est énergiquement agité au moyen d'une plaque ronde métallique perforée mue perpendiculairement à son plan au moyen d'un vibreur fournissant une fréquence d'environ 100 Hz et une amplitude de l'ordre du millimètre.

On introduit ensuite, toujours sous agitation, le ou les métaux sous forme d'une poudre de granulométrie voisine de 150 μm et on chauffe le mélange à 100°C pendant 10 min. On introduit ensuite la quantité voulue de potassium et on porte la température à 160°C pendant 2 h toujours sous agitation. On laisse ensuite refroidir à température ambiante, sous agitation.

La suspension de catalyseur, homogène, est conservée sous atmosphère d'azote.

On a ainsi préparé les catalyseurs dont les compositions figurent au tableau en annexe. Les catalyseurs des exemples 1 à 3 sont comparatifs.

## B) Dimérisation du propylène

On a opéré en discontinu dans un autoclave préalablement rincé au moyen d'une coupe d'hydrocarbures saturés en $C_{12}$-$C_{14}$ et séché à l'azote.

On a introduit dans le réacteur la suspension de catalyseur puis du propylène liquide et on a, sous agitation, chauffé le milieu réactionnel jusqu'à une température de 180-185°C que l'on a maintenue pendant un temps également prédéterminé. La pression dans le réacteur atteint assez rapidement une valeur maximale puis décroit ensuite régulièrement.

Le milieu réactionnel est analysé, après dégazage pour éliminer le propylène n'ayant pas réagi, par chromatographie en phase gazeuse. On a ainsi déterminé la quantité totale de dimères du propylène formés qui, rapportée à la quantité totale de propylène mis à réagir exprime le taux de conversion. La quantité de méthyl-4-pentène-1 formé rapportée à la quantité totale de dimères obtenus exprime la sélectivité de la réaction. Le produit du taux de conversion par le taux de sélectivité exprime donc le rendement de la réaction en méthyl-1-pentène-1 par rapport au propylène mis à réagir.

On a fait figurer au tableau en annexe,
- la masse de propylène mise à réagir, Mp, exprimée en grammes,
- le rapport molaire propylène/potassium, P/K,
- la pression maximale dans le réacteur, Pr1, exprimée en bars,
- la pression Pr2 dans le réacteur en fin d'essai, exprimée en bars,
- la durée totale de la réaction, t, exprimée en minutes,
- le taux de conversion C, exprimé en %,
- le taux de sélectivité S, exprimé en %,
- le rendement R en méthyl-4-pentène-1, exprimé en %.

Les exemples 1 à 3 sont fournis à titre comparatif.

TABLEAU

| Exemple | Métal (M) | K/K₂CO₃ | Rapport pondéral | | Cu/K | Mp | P/K | Pr1 | Pr2 | t | C | S | R |
|---|---|---|---|---|---|---|---|---|---|---|---|---|---|
| | | | (M)/K | Cu/(M) | | | | | | | | | |
| 1 | -- | 0,054 | -- | 0 | 1,48 | 93,8 | 72,2 | 136 | 66 | 140 | 24,7 | 84,3 | 20,0 |
| 2 | Co | 0,050 | 1,53 | 0 | 0 | 93,6 | 70,7 | 133 | 85 | 165 | 23,3 | 80,2 | 18,7 |
| 3 | Co | 0,056 | 0,63 | 1,11 | 0,7 | 94,6 | 71,1 | 115 | 50 | 170 | 25,9 | 86,9 | 22,5 |
| 4 | Mn | 0,054 | 1,60 | 0 | 0 | 96,1 | 78,5 | 170 | 60 | 120 | 37,1 | 84,9 | 31,5 |
| 5 | Mn | 0,050 | 2,50 | 0 | 0 | 95,5 | 122 | 148 | 63 | 180 | 33,3 | 88,6 | 29,5 |
| 6 | Mn | 0,049 | 0,82 | 0,98 | 0,8 | 92,7 | 75,7 | 105 | 50 | 140 | 39,3 | 85,3 | 33,5 |
| 7 | Cd | 0,048 | 0,91 | 1,02 | 0,92 | 95,6 | 82,2 | 130 | 50 | 150 | 43,9 | 84 | 36,9 |

**Revendications**

**Revendications pour les Etats contractants suivants : AT, BE, CH, DE, FR, GB, GR, IT, LI, LU, NL, SE**

1. Catalyseur de dimérisation du propylène en méthyl-4-pentène-1 comprenant du potassium (K) et du carbonate de potassium ($K_2CO_3$), caractérisé en ce qu'il contient en outre un métal (M) choisi parmi le manganèse et le cadmium.

**2.** Catalyseur selon la revendication 1, caractérisé en ce qu'il comprend en outre du cuivre.

**3.** Catalyseur selon l'une des revendications 1 et 2, caractérisé en ce que le rapport pondéral (M)/K est compris entre 0,4 et 5.

**4.** Catalyseur selon l'une des revendications 2 et 3, caractérisé en ce que le rapport pondéral Cu/(M) est au plus égal à 2.

**5.** Catalyseur selon l'une des revendications 1 à 4, caractérisé en ce que le métal (M) et, le cas échéant, le cuivre sont à l'état de poudre ayant une granulométrie comprise entre 30 et 300 $\mu$m.

**6.** Catalyseur selon l'une des revendications 1 à 5, caractérisé en ce qu'il se présente sous la forme d'une suspension dans au moins un liquide inerte.

**7.** Procédé de préparation d'un catalyseur conforme à l'une des revendications 1 à 6 consistant à opérer en présence d'un liquide inerte à haut point d'ébullition selon la séquence d'étapes suivantes :
- dans une première étape on réalise une suspension de carbonate de potassium dans le liquide inerte,
- dans une deuxième étape on introduit dans le milieu réactionnel fortement agité le ou les métaux (M), et le cas échéant du cuivre, sous forme pulvérulente,
- dans une troisième étape, après avoir porté le milieu réactionnel à une température supérieure à la température de fusion du potassium, on introduit le potassium sous forte agitation,
- dans une quatrième étape on porte le milieu réactionnel à une température comprise entre 140 et 180°C pendant une durée comprise entre 1 et 3 heures, puis on le laisse refroidir à température ambiante.

**8.** Procédé de dimérisation du propylène en méthyl-4-pentène-1, caractérisé en ce qu'il consiste à utiliser comme catalyseur au moins un catalyseur conforme à l'une des revendications 1 à 6.

**9.** Procédé selon la revendication 8, caractérisé en, ce que la température de la dimérisation est comprise entre 150 et 220°C.

**10.** Procédé selon l'une des revendications 8 et 9, caractérisé en ce que le rapport molaire propylène/potassium est compris entre 50 et 300.

**Revendications pour les l'Etat contractant suivant : ES**

**1.** Procédé de préparation d'un catalyseur de dimérisation du propylène en méthyl - 4 - pentène - 1 comprenant du potassium (K) et du carbonate de potassium (K$_2$CO$_3$), caractérisé en ce qu'il consiste à opérer en présence d'un liquide inerte à haut point d'ébullition selon la séquence d'étapes suivantes afin que ledit catalyseur contienne en outre un métal (M) choisi parmi le manganèse et le cadmium et le cas échéant du cuivre :
- dans une première étape on réalise une suspension de carbonate de potassium dans le liquide inerte,
- dans une deuxième étape on introduit dans le milieu réactionnel fortement agité le ou les métaux (M) et le cas échéant du cuivre, sous forme pulvérulente,
- dans une troisième étape, après avoir porté le milieu réactionnel à une température supérieure à la température de fusion du potassium, on introduit le potassium sous forte agitation,
- dans une quatrième étape on porte le milieu réactionnel à une température comprise entre 140 et 180°C pendant une durée comprise entre 1 et 3 heures, puis on laisse refroidir à température ambiante.

**2.** Procédé suivant la revendication 1, caractérisé en ce que le rapport pondéral (M)/K est compris entre 0,4 et 5.

**3.** Procédé suivant l'une des revendications 1 et 2, caractérisé en ce que le rapport pondéral Cu/(M) est

EP 0 291 411 B1

au plus égal à 2.

**4.** Procédé suivant l'une des revendications 1 à 3, caractérisé en ce que le métal (M) et, le cas échéant, le cuivre sont à l'état de poudre ayant une granulométrie comprise entre 30 et 300 $\mu$m.

**5.** Procédé suivant l'une des revendications 1 à 4, caractérisé en ce que le catalyseur se présente sous la forme d'une suspension dans au moins un liquide inerte.

**6.** Procédé de dimérisation du propylène en méthyl - 4 - pentène - 1, caractérisé en ce qu'il consiste à utiliser le catalyseur obtenu selon l'une des revendications 1 à 5.

**7.** Procédé suivant la revendication 6, caractérisé en ce que la température de dimérisation est comprise entre 150 et 220°C.

**8.** Procédé suivant l'une des revendications 6 et 7, caractérisé en ce que le rapport molaire propylène/potassium est compris entre 50 et 300.

**Claims**
**Claims for the following Contracting States : AT, BE, CH, DE, FR, GB, GR, IT, LI, LU, NL, SE**

**1.** Catalyst for the dimerisation of propylene to 4-methyl-1-pentene, comprising potassium (K) and potassium carbonate ($K_2CO_3$), characterised in that it additionally contains a metal (M) chosen from manganese and cadmium.

**2.** Catalyst according to Claim 1, characterised in that it additionally comprises copper.

**3.** Catalyst according to one of Claims 1 and 2, characterised in that the (M)/K weight ratio is between 0.4 and 5.

**4.** Catalyst according to one of Claims 2 and 3, characterised in that the Cu/(M) weight ratio does not exceed 2.

**5.** Catalyst according to one of Claims 1 to 4, characterised in that the metal (M) and, where appropriate, copper are in the state of powder having a particle size of between 30 and 300 $\mu$m.

**6.** Catalyst according to one of Claims 1 to 5, characterised in that it takes the form of a suspension in at least one inert liquid.

**7.** Process for preparing a catalyst according to one of Claims 1 to 6, which consists in working in the presence of an inert, high boiling point liquid according to the following sequence of steps:
- in a first step, a suspension is produced of potassium carbonate in the inert liquid,
- in a second step, the metal or metals (M) and, where appropriate, copper is/are introduced in powder form into the vigorously agitated reaction medium,
- in a third step, after the reaction medium has been brought to a temperature above the melting point of potassium, potassium is introduced with vigorous agitation,
- in a fourth step, the reaction medium is brought to a temperature of between 140 and 180°C for a period of between 1 and 3 hours, and then allowed to cool to room temperature.

**8.** Process for the dimerisation of propylene to 4-methyl-1-pentene, characterised in that it consists in using as a catalyst at least one catalyst according to one of Claims 1 to 6.

**9.** Process according to Claim 8, characterised in that the dimerisation temperature is between 150 and 220°C.

**10.** Process according to one of Claims 8 and 9, characterised in that the propylene/potassium molar ratio is between 50 and 300.

**Claims for the following Contracting State : ES**

7

1. Process for preparing a catalyst for the dimerisation of propylene to 4-methyl-1-pentene, comprising potassium (K) and potassium carbonate ($K_2CO_3$), characterised in that it consists in working in the presence of an inert, high boiling point liquid according to the following sequence of steps in order that the said catalyst additionally contains a metal (M) chosen from manganese and cadmium and, where appropriate, copper:
   - in a first step, a suspension is produced of potassium carbonate in the inert liquid,
   - in a second step, the metal or metals (M) and, where appropriate, copper is/are introduced in powder form into the vigorously agitated reaction medium,
   - in a third step, after the reaction medium has been brought to a temperature above the melting point of potassium, potassium is introduced with vigorous agitation,
   - in a fourth step, the reaction medium is brought to a temperature of between 140 and 180°C for a period of between 1 and 3 hours, and then allowed to cool to room temperature.

2. Process according to Claim 1, characterised in that the (M)/K weight ratio is between 0.4 and 5.

3. Process according to one of Claims 1 and 2, characterised in that the Cu/(M) weight ratio does not exceed 2.

4. Process according to one of Claims 1 to 3, characterised in that the metal (M) and, where appropriate, copper are in the state of powder having a particle size of between 30 and 300 $\mu$m.

5. Process according to one of Claims 1 to 4, characterised in that the catalyst takes the form of a suspension in at least one inert liquid.

6. Process for the dimerisation of propylene to 4-methyl-1-pentene, characterised in that it consists in using the catalyst obtained according to one of Claims 1 to 5.

7. Process according to Claim 6, characterised in that the dimerisation temperature is between 150 and 220°C.

8. Process according to one of Claims 6 and 7, characterised in that the propylene/potassium molar ratio is between 50 and 300.


**Patentansprüche**

**Patentansprüche für folgende Vertragsstaaten : AT, BE, CH, DE, FR, GB, GR, IT, LI, LU, NL, SE**

1. Katalysator zur Dimerisierung von Propylen in 4-Methylpenten-1, der Kalium (K) und Kaliumcarbonat ($K_2CO_3$) enthält, dadurch gekennzeichnet, daß er desweiteren ein Metall (M) enthält, das aus Mangan und Cadmium ausgewählt ist.

2. Katalysator nach Anspruch 1, dadurch gekennzeichnet, daß er außerdem Kupfer enthält.

3. Katalysator nach einem der Ansprüche 1 und 2, dadurch gekennzeichnet, daß das Gewichtsverhältnis (M)/K zwischen 0,4 und 5 liegt.

4. Katalysator nach einem der Ansprüche 2 und 3, dadurch gekennzeichnet, daß das Gewichtsverhältnis Cu/(M) höchstens 2 beträgt.

5. Katalysator nach einem der Ansprüche 1 bis 4, dadurch gekennzeichnet, daß das Metall (M) und gegebenenfalls das Kupfer in Form eines Pulvers einer Körnergröße zwischen 30 und 300 $\mu$m vorliegen.

6. Katalysator nach einem der Ansprüche 1 bis 5, dadurch gekennzeichnet, daß er in Form einer Suspension in wenigstens einer inerten Flüssigkeit vorliegt.

7. Verfahren zur Herstellung eines Katalysators nach einem der Ansprüche 1 bis 6, bei dem in Gegenwart einer inerten Flüssigkeit mit hohem Siedepunkt folgende Schritte durchgeführt werden:
   - in einem ersten Schritt stellt man eine Kaliumcarbonatsuspension in der inerten Flüssigkeit her,

- in einem zweiten Schritt setzt man dem Reaktionsgemisch unter starkem Rühren das oder die Metalle (M), und gegebenenfalls Kupfer, in Pulverform zu,
- in einem dritten Schritt, nachdem man das Reaktionsgemisch auf eine Temperatur über den Schmelzpunkt des Kaliums gebracht hat, fügt man das Kalium unter starkem Rühren hinzu,
- in einem vierten Schritt bringt man das Reaktionsgemisch auf eine Temperatur zwischen 140 und 180°C während einer Dauer von einer bis drei Stunden und läßt es anschließend auf Raumtemperatur abkühlen.

8. Verfahren zur Dimerisierung von Propylen in 4-Methylpenten-1, dadurch gekennzeichnet, daß als Katalysator wenigstens ein Katalysator nach einem der Ansprüche 1 bis 6 verwendet wird.

9. Verfahren nach Anspruch 8, dadurch gekennzeichnet, daß die Temperatur der Dimerisierung zwischen 150 und 220°C liegt.

10. Verfahren nach einem der Ansprüche 8 und 9, dadurch gekennzeichnet, daß das Molverhältnis Propylen/Kalium zwischen 50 und 300 liegt.

**Patentansprüche für folgenden Vertragsstaat : ES**

1. Verfahren zur Herstellung eines Katalysators zur Dimerisierung von Propylen in 4-Methylpenten-1, der Kalium (K) und Kaliumcarbonat ($K_2CO_3$) enthält, dadurch gekennzeichnet, daß in Gegenwart einer inerten Flüssigkeit mit hohem Siedepunkt folgende Schritte durchgeführt werden, damit der Katalysator desweiteren ein Metall (M) enthält, das aus Mangan und Cadmium und gegebenenfalls Kupfer ausgewählt ist:
   - in einem ersten Schritt stellt man eine Kaliumcarbonatsuspension in der inerten Flüssigkeit her,
   - in einem zweiten Schritt setzt man dem Reaktionsgemisch unter starkem Rühren das oder die Metalle (M), und gegebenenfalls Kupfer, in Pulverform zu,
   - in einem dritten Schritt, nachdem man das Reaktionsgemisch auf eine Temperatur über den Schmelzpunkt des Kaliums gebracht hat, fügt man das Kalium unter starkem Rühren hinzu,
   - in einem vierten Schritt bringt man das Reaktionsgemisch auf eine Temperatur zwischen 140 und 180°C während einer Dauer von einer bis drei Stunden und läßt es anschließend auf Raumtemperatur abkühlen.

2. Verfahren nach Anspruch 1, dadurch gekennzeichnet, daß das Gewichtsverhältnis (M)/K zwischen 0,4 und 5 liegt.

3. Verfahren nach einem der Ansprüche 1 und 2, dadurch gekennzeichnet, daß das Gewichtsverhältnis Cu/(M) höchstens 2 beträgt.

4. Verfahren nach einem der Ansprüche 1 bis 3, dadurch gekennzeichnet, daß das Metall (M) und gegebenenfalls das Kupfer in Form eines Pulvers einer Körnergröße zwischen 30 und 300 μm vorliegen.

5. Verfahren nach einem der Ansprüche 1 bis 4, dadurch gekennzeichnet, daß der Katalysator in Form einer Suspension in wenigstens einer inerten Flüssigkeit vorliegt.

6. Verfahren zur Dimerisierung von Propylen in 4-Methylpenten-1, dadurch gekennzeichnet, daß der erhaltene Katalysator nach einem der Ansprüche 1 bis 5 verwendet wird.

7. Verfahren nach Anspruch 6, dadurch gekennzeichnet, daß die Temperatur der Dimerisierung zwischen 150 und 220°C liegt.

8. Verfahren nach einem der Ansprüche 6 und 7, dadurch gekennzeichnet, daß das Molverhältnis Propylen/Kalium zwischen 50 und 300 liegt.